# EUROPEAN PATENT APPLICATION

(11) **EP 4 353 741 A1**
(43) Date of publication of application: **17.04.2024**
(21) Application number: 22201705.5
(22) Date of filing: 14.10.2022
(51) Int. Cl.: C07K 14/47, A61K 35/17, C07K 14/705, C12N 5/0783

(54) **DOUBLE KNOCKOUT NATURAL KILLER CELLS**

(71) Applicant: ONK Therapeutics Limited, Co. Galway H91 V6KV (IE)
(72) Inventor: The designation of the inventor has not yet been filed
(74) Representative: Hughes, Sean David

(57) **Abstract**

NK cells and NK cell lines are modified to increase their cytotoxicity, proliferation, metabolic profile and persistence, wherein the cells and compositions thereof have a use in the treatment of cancer. Production of modified NK cells and NK cell lines is via genetic modification to knockout expression of both CISH and NKG2A genes

## Description

### Introduction

The present invention relates to the modification of natural killer (NK) cells and NK cell lines to produce derivatives thereof with a more cytotoxic phenotype. Furthermore, the present invention relates to methods of producing modified NK cells and NK cell lines, compositions containing the cells and cell lines and uses of said cells, lines and compositions in the treatment of cancer.

### Background to the Invention

Typically, immune cells require a target cell to present antigen via major histocompatibility complex (MHC) before triggering an immune response resulting in the death of the target cell. This allows cancer cells not presenting MHC class I to evade the majority of immune responses.

NK cells, however, are able to recognize cancer cells in the absence of MHC class I expression. Hence they perform a critical role in the body's defence against cancer.

On the other hand, in certain circumstances, cancer cells demonstrate an ability to dampen the cytotoxic activity of NK cells, through expression of ligands that bind inhibitory receptors on the NK cell membrane. Resistance to cancer can involve a balance between these and other factors.

Cytotoxicity, in this context, refers to the ability of immune effector cells, e.g. NK cells, to induce cancer cell death, e.g. by releasing cytolytic compounds or by binding receptors on cancer cell membranes and inducing apoptosis of said cancer cells. Cytotoxicity is affected not only by signals that induce release of cytolytic compounds but also by signals that inhibit their release. An increase in cytotoxicity will therefore lead to more efficient killing of cancer cells, with less chance of the cancer cell dampening the cytotoxic activity of the NK, as mentioned above.

Cytokine-inducible SH2-containing protein (CISH) expression is induced by certain growth cytokines and is a key negative regulator of interleukin-15 (IL-15) signalling in natural killer (NK) cells. CISH expression is associated with limited cell expansion and decreased cytotoxic activity against multiple cancer cell lines when maintained at low cytokine concentrations. As such, knocking out CISH expression by deletion of the gene in NK cells has recently been reported as beneficial for NK cell cytotoxicity against cancer cells (Zhu et al. 2020 "Metabolic Reprograming via Deletion of CISH in Human iPSC-Derived NK Cells Promotes In Vivo Persistence and Enhances Antitumor Activity" Cell: Vol. 27(2): pp. 224-237).

There nevertheless exists a need for alternative and preferably improved cell-based therapies with improved cytotoxicity and persistence, particularly against cancer cells. In addition, it is desirable to achieve rapid and highly efficient transfection of the cells such that they can be taken to the clinic in a modified, therapeutic form with greater ease.

An object of the present invention is to provide NK cells and NK cell lines that target cancer cells with a more cytotoxic and/or persistent phenotype. A further object is to provide methods for producing modified NK cells and NK cell lines, compositions containing the cells or cell lines and uses of such in therapy, specifically the treatment of cancers. More particular embodiments aim to provide treatments for identified cancers. Specific embodiments aim at combining two or more modifications of NK cells and NK cell lines to enhance the cytotoxicity and persistence of the modified cells.

### Summary of the Invention

There are provided herein modified NK cells and NK cell lines with a more cytotoxic phenotype, and methods of making the cells and cell lines. Also provided are compositions of modified NK cells and NK cell lines, and uses of said compositions for treating cancer. Cytotoxicity in this context, as above, refers to the killing of cancer cells.

The invention provides NK cells and NK cell lines that have been modified to have reduced function of both CISH and NKG2A.

According to the invention, there are further provided methods of treating cancer, e.g. blood cancer, using the modified NK cells and cell lines, both generally and specifically, wherein the modified NK cells and lines are engineered to have reduced function of CISH and NKG2A and, optionally, in addition to lack expression of one or more checkpoint inhibitory receptors, to express one or more TRAIL ligands, to express one or more chimeric antigen receptors, to express one or more growth-promoting cytokines and/or to express one or more Fc receptors.

Diseases particularly treatable according to the invention include cancers, solid cancers and blood cancers. Tumours and cancers in humans in particular can be treated. References to tumours herein include references to neoplasms.

### Details of the Invention

Accordingly, the present invention provides a natural killer (NK) cell or NK cell line that has been modified to have reduced function of both CISH and NKG2A.

Together, the NK cells and NK cell lines of the invention will also be referred to as the NK cells (unless the context requires otherwise). Preferably, the NK cells are human NK cells.

As described in detail below in examples, NK cells and NK cell lines have been genetically modified so as to increase their cytotoxic activity, particularly against cancer. It is therefore preferable that the modification is to knock down or knock out expression of CISH and NKG2A.

It is preferred that the modified NK cells comprise no further modifications resulting in reduced expression of TIGIT or TGFBRII. Preferred cells of the invention have a normal or baseline expression of both TIGIT and TGFBRII, these genes not having been genetically modified.

Preferably still, the CISH / NKG2A double knockout (DKO) is performed in an NK cell or cell line without any further genetic knockdowns or knockouts. That is to say, the NK cells being modified have no existing genetic knockdowns or knockouts, nor are there additional genetic knockdowns or knockouts introduced at the same time or after the CISH / NKG2A DKO is introduced. As described elsewhere herein, the DKO NK cells of the invention may have one or more genes knocked in.

It is specifically preferred that other NK cell inhibitory receptors are not knocked out in the same NK cell / NK cell line comprising the CISH / NKG2A DKO. This is because the synergy achieved through the DKO (explained below) may otherwise be masked.

As such, it is preferred that the DKO NK cells of the invention have normal / baseline expression of all other NK cell inhibitory receptors, excluding CISH and NKG2A.

Preferably, CISH function and/or expression is reduced by at least 50%, at least 75%, at least 90%, at least 95%, more preferably at least 99%, compared to the same NK cell or NK cell line without the modification (wildtype NK cell).

Preferably, NKG2A function and/or expression is reduced by at least 50%, at least 75%, at least 90%, at least 95%, more preferably at least 99%, compared to the same NK cell or NK cell line without the modification (wildtype NK cell).

It is particularly preferred that NK cells according to the invention have both CISH and NKG2A expression knocked out. Genetic knockout of CISH and/or NKG2A is preferably by CRISPR gene editing, e.g. as achieved in examples of the invention.

A preferred feature of the invention is to provide modified NK cells and NK cell lines with an increased intrinsic capacity for rapid growth and proliferation in culture. This can be achieved, for example, by transfecting the cells to overexpress growth-inducing cytokines IL-2 and/or IL-15, very suitably soluble forms thereof (sIL-15). Moreover, this optional alteration provides a cost-effective alternative to replenishing the growth medium with cytokines on a continuous basis. These cells can be used as intermediates to prepare significant quantities of cells for subsequent processing (e.g. to reduce their divisional capacity) prior to therapeutic application.

The modified NK cells of the invention may thus additionally be modified to express IL-15 or sIL-15, or, in cases where the NK cells already express a certain amount of IL-15, they may be modified to overexpress IL-15 or sIL-15.

A specific embodiment of the invention provides a DKO NK cell also genetically modified to express sIL-15, for human cancer therapy.

A significant advantage of the invention is in the way the NK cell modifications work together to provide a highly effective, persistent and cytotoxic effector cell. While knocking out CISH expression in NK cells results in increased proliferation and cytokine production, the resulting increase in IFNy production can significantly increase HLA-E expression on the targeted cancer. Surprisingly, however, it is shown herein that additionally knocking out NKG2A expression in the NK cells increases their cytotoxicity potently. In fact, the CISH / NKG2A DKO is synergistic, in that the CISH KO not only restores any loss in cytotoxicity from the NKG2A KO, but it boosts the cytotoxicity significantly beyond what is seen in a CISH KO cell. This unexpected synergy from the combination of knocking out both CISH and NKG2A in NK cells, therefore, provides an effector NK cell that is more cytotoxic towards its target (e.g., cancer cell), more persistent and more metabolically resilient. Mechanistically, without the NKG2A KO, the CISH KO-induced upregulation of HLA-E expression on the cancer would, in time, dampen NK cell cytotoxicity through its binding to the NKG2A receptor.

Preferably, the NK cells are derived from cord blood (CB-NK cells). These specific primary NK cells are mature NK cells that have undergone their 'education'. A significant benefit of using CB-NK cells is that they are able to tolerate knocking out expression of the classical inhibitory receptor NKG2A without significant alteration to their primary cytotoxic functions. A further advantage to this characteristic is that knocking out CISH in NK cells results in increased IFNy production; this in turn promotes HLA-E expression on cancer cells (a ligand for NKG2A). As such, without a concurrent NKG2A KO, the CISH KO NK cells would inadvertently increase the ability of cancer cells to downregulate NK cell cytotoxicity.

In other embodiments of the invention, DKO NK cells are provided that also express or overexpress a TRAIL ligand. Preferably, the TRAIL ligand is a mutant (variant) TRAIL ligand. The resulting NK cells exhibit increased binding to TRAIL receptors and, as a result, increased cytotoxicity against cancers, especially solid cancers, in particular ovarian, breast and colorectal cancers, and blood cancers, in particular leukemias. The NK cells with this combined activity may also be effective in reducing cancer metastases.

The TRAIL mutants / variants preferably have lower affinity (or in effect no affinity) for 'decoy' receptors, compared with the binding of wild type TRAIL to decoy receptors. Such decoy receptors represent a class of TRAIL receptors that bind TRAIL ligand but do not have the capacity to initiate cell death and, in some cases, act to antagonize the death signaling pathway. Mutant / variant TRAIL ligands may be prepared according to WO 2009/077857.

The mutants / variants may separately have increased affinity for TRAIL receptors, e.g. DR4 and DR5. Wildtype TRAIL is typically known to have a KD of >2 nM for DR4, >5 nM for DR5 and >20 nM for the decoy receptor DcR1 (WO 2009/077857; measured by surface plasmon resonance), or around 50 to 100 nM for DR4, 1 to 10 nM for DR5 and 175 to 225 nM for DcR1 (Truneh, A. et al. 2000; measured by isothermal titration calorimetry and ELISA). Therefore, an increased affinity for DR4 is suitably defined as a KD of <2 nM or <50 nM, respectively, whereas an increased affinity for DR5 is suitably defined as a KD of <5 nM or <1 nM, respectively. A reduced affinity for decoy receptor DcR1 is suitably defined as a KD of >50 nM or >225 nM, respectively. In any case, an increase or decrease in affinity exhibited by the TRAIL variant/mutant is relative to a baseline affinity exhibited by wildtype TRAIL. The affinity is preferably increased at least 10%, at least 25%, at least 50%, at least 100%, more preferably at least 1000%, compared with that exhibited by wildtype TRAIL.

The TRAIL variant preferably has an increased affinity for DR5 as compared with its affinity for DR4, DcR1 and DcR2. Preferably, the affinity is at least 1.5-fold, 2-fold, 5-fold, 10-fold, 100-fold, or even 1,000-fold or greater for DR5 than for one or more of DR4, DcR1 and DcR2. More preferably, the affinity is at least 1.5-fold, 2-fold, 5-fold, 10-fold, 100-fold, or even 1,000-fold or greater for DR5 than for at least two, and preferably all, of DR4, DcR1 and DcR2.

The TRAIL variant preferably has an increased affinity for one or both of DR4 and DR5 as compared with its affinity for wildtype TRAIL. Preferably, the affinity is at least 1.5-fold, 2-fold, 5-fold, 10-fold, 100-fold, or even 1,000-fold or greater for DR4 and/or DR5 than for wildtype TRAIL.

Further specific embodiments comprise a NK cell expressing a mutant TRAIL ligand that has reduced or no affinity for TRAIL decoy receptors. Further specific embodiments comprise a NK cell expressing a mutant TRAIL ligand that has reduced or no affinity for TRAIL decoy receptors and increased affinity for DR4 and/or DR5.

Binding affinity may be measured according to any suitable method known in the art. Preferably, binding affinity is measured using surface plasmon resonance, isothermal titration calorimetry or ELISA.

In certain embodiments, the TRAIL variant comprises at least one amino acid substitution at a position selected from the group consisting of 131, 149, 159, 160, 189, 191, 193, 195, 199, 200, 201, 203, 204, 212, 213, 214, 215, 218, 240, 251, 261, 264, 266, 267, 269, and 270.

In certain embodiments, the TRAIL variant comprises at least one substitution selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, and H270D.

In certain embodiments, the TRAIL variant comprises at least two substitutions selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, and H270D.

In certain embodiments, the TRAIL variant comprises at least three substitutions selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, and H270D.

In certain embodiments, amino acid substitution of the TRAIL variant is selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, H270D, T214R / E195R, T214R / D269H, Y189A / Q193S / N199V / K201R / Y213W / S215D, Y213W / S215D, N199R / K201H, N199H / K201R, G131R / N199R / K201H, G131R / N199R / K201H / R149I / S159R / S215D, G131R / R149I / S159R / S215D, G131R / N199R / K201H / R149I / S159R / S215D, G131R / D218H, Y189Q / R191K / Q193R / H264R / I266L / D267Q, T261L / G160E, T261L / H270D, T261L / G160E / H270D, and T261L / G160E / H270D / T200H (use of "/" indicates multiple amino acid substitutions).

In certain embodiments, amino acid substitution of the TRAIL variant is selected based on the variant having an increased affinity for DR5; a substitution of this kind may be selected from the group consisting of D269H, E195R, T214R, D269H / E195R, T214R / E195R, T214R / D269H, N199V, Y189A / Q193S / N199V / K201R / Y213W / S215D, Y213W / S215D, D269A and Y240A.

In certain embodiments, amino acid substitution of the TRAIL variant is selected based on the variant having an increased affinity for DR4; a substitution of this kind may be selected from the group consisting of G131R, G131K, R149I, R149M, R149N, R149K, S159R, Q193H, W193K, N199R, N199R / K201H, N199H / K201R, G131R / N199R / K201H, G131R / N199R / K201H, G131R / N199R / K201H / R149I / S159R / S215D, G131R / R149I / S159R / S215D, G131R / D218H, K201R, K201H, K204E, K204D, K204L, K204Y, K212R, S215E, S215H, S215K, S215D, D218H, K251D, K251E, K251Q and Y189Q / R191K / Q193R / H264R / I266L / D267Q.

In certain embodiments, amino acid substitution of the TRAIL variant is selected based on the variant having a decreased affinity for TRAIL decoy receptors; a substitution of this kind may be selected from the group consisting of T261L, H270D, T200H, T261L / G160E, T261L / H270D, T261L / G160E / H270D, T261L / G160E / H270D / T200H, D203A and D218A.

Treatment of a cancer using modified NK cells expressing TRAIL or a TRAIL variant is optionally enhanced by administering to a patient an agent capable of upregulating expression of TRAIL death receptors on cancer cells. This agent may be administered prior to, in combination with or subsequently to administration of the modified NK cells. It is preferable, however, that the agent is administered prior to administering the modified NK cells. The agent upregulates expression of DR5 on cancer cells. The agent may optionally be a chemotherapeutic medication, e.g. a proteasome inhibitor, one of which is Bortezomib, and administered in a low dose capable of upregulating DR5 expression on the cancer. Other examples of DR5-inducing agents include Gefitinib, Piperlongumine, Doxorubicin, Alpha-tocopheryl succinate and HDAC inhibitors.

In certain embodiments of the invention, DKO NK cells are provided that are further modified so as to have reduced or absent function of a checkpoint inhibitory receptor. NK cells may be produced that have one or more checkpoint inhibitory receptor genes knocked down/out. Preferably, these receptors are specific checkpoint inhibitory receptors. Preferably still, these checkpoint inhibitory receptors are one or more or all of TIGIT, CD96 (TACTILE), CD152 (CTLA4), CD223 (LAG-3), CD279 (PD-1), CD328 (SIGLEC7), SIGLEC9 and/or TIM-3. In other embodiments, NK cells are provided in which one or more inhibitory receptor signaling pathways are knocked out or exhibit reduced function - the result again being reduced or absent inhibitory receptor function. For example, signaling pathways mediated by SHP-1, SHP-2 and/or SHIP are knocked out by genetic modification of the cells.

It is preferred to reduce function of checkpoint inhibitory receptors over other inhibitory receptors, due to the expression of the former following NK cell activation. The normal or 'classical' inhibitory receptors, such as the majority of the KIR family, NKG2A and LIR-2, bind MHC class I and are therefore primarily involved in reducing the problem of self-targeting. Preferably, therefore, checkpoint inhibitory receptors are knocked out. Reduced or absent function of these receptors according to the invention prevents cancer cells from suppressing immune effector function (which might otherwise occur if the receptors were fully functional). Thus, a key advantage of these embodiments of the invention lies in NK cells that are less susceptible to suppression of their cytotoxic activities by cancer cells; as a result, they are useful in cancer treatment.

As used herein, references to inhibitory receptors generally refer to a receptor expressed on the plasma membrane of an immune effector cell, e.g. a NK cell, whereupon binding its complementary ligand resulting intracellular signals are responsible for reducing the cytotoxicity of said immune effector cell. These inhibitory receptors are expressed during both 'resting' and 'activated' states of the immune effector cell and are often associated with providing the immune system with a 'self-tolerance' mechanism that inhibits cytotoxic responses against cells and tissues of the body. An example is the inhibitory receptor family 'KIR' which are expressed on NK cells and recognize MHC class I expressed on healthy cells of the body.

Also as used herein, checkpoint inhibitory receptors are usually regarded as a subset of the inhibitory receptors above. Unlike other inhibitory receptors, however, checkpoint inhibitory receptors are expressed at higher levels during prolonged activation and cytotoxicity of an immune effector cell, e.g. a NK cell. This phenomenon is useful for dampening chronic cytotoxicity at, for example, sites of inflammation. Examples include the checkpoint inhibitory receptors PD-1, CTLA-4 and CD96, all of which are expressed on NK cells.

It is preferred in the invention not to reduce the function of inhibitory receptors that bind MHC class I.

The modified cells of the invention may also express a chimeric antigen receptor (CAR). The membrane-bound CAR typically comprises a targeting sequence (commonly an antibody-derived single-chain fragment (scFv)) and usually a hinge (to overcome steric hindrance issues), a spacer, a membrane-spanning element and a signalling endodomain.

The CAR preferably comprises a targeting region that binds an antigen selected from PD-L1, VEGF, VEGFR2, PD-1, MUC-1, CLL-1, TIM-3, CD19, EGFR, CD38, GD2, SLAMF7, CTLA4, CCR4, CD20, PDGFRα, HER2, CD33, CD30, CD22, CD79B, Nectin-4 and TROP2.

The CAR preferably comprises a targeting region that binds an antigen selected from HER2, TIM-3, MUC-1, CD38, CD96, CLL-1, SLAMF7 and CD19. Most preferably, the CAR binds an antigen selected from HER2, CD38 and MUC-1.

Examples of sequences that are known to bind aberrantly glycosylated MUC-1 can be used as the targeting sequence, such sequences including 5E5, SM3 and HMFG2, and are suitably incorporated in a CAR of the invention; preferably the CAR comprises the HMFG2 sequence. Nevertheless, further sequences for targeting aberrantly glycosylated MUC-1 may be identified through screening methods known in the prior art, wherein high affinity sequences can be used to produce CAR-NK cells targeting MUC-1.

Examples of sequences that are known to bind CD38 can be used as the targeting sequence, such sequences including daratumumab, isatuximab and those disclosed in WO 2018/104562, and are suitably incorporated in a CAR of the invention. It is preferred that the CD38 CAR has reduced affinity for CD38, compared to the affinity of daratumumab for CD38. Preferably, this reduced affinity is by at least 10%, at least 25%, more preferably at least 45%, compared to the affinity of daratumumab. Preferably, also, this reduced affinity is no more than 90%, no more than 75%, more preferably no more than 55%, compared to the affinity of daratumumab.

The CARs used in the NK cells of the invention may comprise or be linked to one or more NK cell costimulatory domains, e.g. CD28, CD134 / OX40, 4-1BB / CD137, CD3zeta / CD247, DAP12 or DAP10. Binding of the CAR to its antigen on a target cell thus promotes cytotoxic signals in the modified NK cell.

In an optional embodiment, the DKO NK cells of the invention may be used / administered in combination with an antibody specific for an antigen on a cancer cell.

It is preferred that the DKO NK cells are administered in combination with a antibody targeting an antigen selected from PD-L1, VEGF, VEGFR2, PD-1, MUC-1, CLL-1, TIM-3, CD19, EGFR, CD38, GD2, SLAMF7, CTLA4, CCR4, CD20, PDGFRα, HER2, CD33, CD30, CD22, CD79B, Nectin-4 and TROP2.

Preferably, the antibody used in combination with the DKO NK cells is selected from Atezolizumab, Avelumab, Bevacizumab, Brentuximab, Blinatumomab, Cemiplimab, Cetuximab, Daratumumab, Dinutuximab, Durvalumab, Elotuzumab, Enfortumab, Gemtuzumab, Ibritumomab, Ipilimumab, Inotuzumab, Isatuximab, Mogamulizumab, Necitumumab, Nivolumab, Obinutuzumab, Ofatumumab, Olaratumab, Panitumumab, Pembrolizumab, Pertuzumab, Polatuzumab, Ramucirumab, Rituximab, Sacituzumab, Tositumomab and Trastuzumab.

More preferably, the antibody used in combination with the DKO NK cells is Trastuzumab or Daratumumab

NK cells according to the invention may also be treated or pre-treated to render them incapable of division. This results in further reduced lifetime in circulation in the patient, e.g. in comparison with T cells, further mitigating the risks above, and also with reduced or absent propensity to form tumours in a patient.

NK cells of the invention are for use in therapy, especially in treating cancer in a patient. The cancer is suitably a cancer expressing CD38. Preferably, the cancer is a blood cancer, e.g. myeloma or leukaemia, or a solid cancer.

The DKO NK cells of the invention are preferably used to treat HLA-E expressing cancers.

NK cell resistant cancers in general are well-known in the art (Pardoll, D.M. Immunity (2015) 42:605-606). Sensitivity of cancer cells to NK cell-mediated killing is determined by a number of factors. There exists a balance of positive and negative signals, largely delivered through membrane receptors on NK cells interacting with ligands on cancer cells. It is often the balance of expression of the ligands for these receptors that determines whether a cancer is sensitive or resistant to killing by NK cells (Yokoyama, W.M. Immunol Res (2005) 32:317-325).

Cancer sensitivity to NK cell-mediated cytotoxicity is generally understood as falling into one of the following categories: highly resistant, resistant, sensitive and highly sensitive. In the laboratory, cancer cells can be screened for their sensitivity to NK cell-mediated cytotoxicity through the use of cytotoxicity assays. Each category is then understood as corresponding to the percentage of cancer cells that are killed during exposure to NK cells at a specific effector:target (E:T) ratio and for a specific amount of time.

In examples of the invention, a cancer is said to be highly resistant to NK cell-mediated killing if ≤ 25% of the cancer cells are killed after incubation with NK cells for up to 15 hours at an E:T ratio of up to 5:1. Preferably, the NK cells are KHYG-1 cells.

In examples of the invention, a cancer is said to be resistant to NK cell-mediated killing if ≤ 50% of the cancer cells are killed after incubation with NK cells for up to 15 hours at an E:T ratio of up to 5:1. Preferably, the NK cells are KHYG-1 cells.

In examples of the invention, a cancer is said to be sensitive to NK cell-mediated killing if > 50% of the cancer cells are killed after incubation with NK cells for up to 15 hours at an E:T ratio of up to 5:1. Preferably, the NK cells are KHYG-1 cells.

In examples of the invention, a cancer is said to be highly sensitive to NK cell-mediated killing if ≥ 75% of the cancer cells are killed after incubation with NK cells for up to 15 hours at an E:T ratio of up to 5:1. Preferably, the NK cells are KHYG-1 cells.

In preparing genetically modified NK cells, the modification may occur before the cell has differentiated into an NK cell. For example, pluripotent stem cells (e.g. iPSCs) can be genetically modified then differentiated to produce genetically modified NK cells with increased cytotoxicity.

Optional features of the invention include providing further modifications to the NK cells and NK cell lines described above, wherein, for example, a Fc receptor (which can be CD16, CD32 or CD64, including subtypes and derivatives) is expressed on the surface of the cell. In use, these cells can show increased recognition of antibody-coated cancer cells and improved activation of the cytotoxic response.

Further optional features of the invention include adapting the modified NK cells and NK cell lines to better home to specific target regions of the body. NK cells of the invention may be targeted to specific cancer cell locations. In preferred embodiments for treatment of blood cancers, NK effectors of the invention are adapted to home to bone marrow. Specific NK cells are modified by fucosylation and/or sialylation to home to bone marrow. This may be achieved by genetically modifying the NK cells to express the appropriate fucosyltransferase and/or sialyltransferase, respectively. Increased homing of NK effector cells to tumour sites may also be made possible by disruption of the tumour vasculature, e.g. by metronomic chemotherapy, or by using drugs targeting angiogenesis (Melero et al, 2014) to normalize NK cell infiltration via cancer blood vessels.

The invention additionally provides a composition comprising an NK cell as described above.

The invention further provides a method of making an NK cell as described above, preferably the method comprising:
a) knocking out expression of the CISH gene;
b) knocking out expression of the NKG2A gene; and, optionally,
c) knocking in expression of the IL-15 gene.

Preferably, the CISH gene and NKG2A gene are knocked out simultaneously; if knocked-out sequentially, this can be in either order.

Modified NK cells, NK cell lines and compositions thereof described herein, above and below, are suitable for treatment of cancer, in particular cancer in humans, e.g. for treatment of cancers of blood cells or solid cancers. The NK cells and derivatives thereof are preferably human NK cells. For human therapy, human NK cells are preferably used. The invention also provides methods of treating cancer in humans comprising administering an effective amount of the NK cells or compositions comprising the same.

Various routes of administration will be known to the skilled person to deliver active agents and combinations thereof to a patient in need. Administration of the modified NK cells can be systemic or localized, such as for example via the intraperitoneal route. In other embodiments, active agent is administered more directly. Thus, administration can be directly intratumoural, suitable especially for solid tumours.

NK cells in general are believed suitable for the methods, uses and compositions of the invention. As per cells used in certain examples herein, the NK cell can be a NK cell obtained from a cancer cell line. Advantageously, a NK cell, preferably treated to reduce its tumorigenicity, for example by rendering it mortal and/or incapable of dividing, can be obtained from a blood cancer cell line and used in methods of the invention to treat blood cancer.

To render a cancer-derived NK cell more acceptable for therapeutic use, it is generally treated or pre-treated in some way to reduce or remove its propensity to form tumours in the patient. Specific modified NK cell lines used in examples are safe because they have been rendered incapable of division; they are irradiated and retain their killing ability but die within about 3-4 days. Specific cells and cell lines are hence incapable of proliferation, e.g. as a result of irradiation. Treatments of potential NK cells for use in the methods herein include irradiation to prevent them from dividing and forming a tumour *in vivo* and genetic modification to reduce tumorigenicity, e.g. to insert a sequence encoding a suicide gene that can be activated to prevent the cells from dividing and forming a tumour *in vivo.* Suicide genes can be turned on by exogenous, e.g. circulating, agents that then cause cell death in those cells expressing the gene. A further alternative is the use of monoclonal antibodies targeting specific NK cells of the therapy. CD52, for example, is expressed on KHYG-1 cells and binding of monoclonal antibodies to this marker can result in antibody-dependent cell-mediated cytotoxicity (ADCC) and KHYG-1 cell death.

As discussed in an article published by Suck et al, 2006, cancer-derived NK cells and cell lines are easily irradiated using irradiators such as the Gammacell 3000 Elan. A source of Cesium-137 is used to control the dosing of radiation and a dose-response curve between, for example, 1 Gy and 50 Gy can be used to determine the optimal dose for eliminating the proliferative capacity of the cells, whilst maintaining the benefits of increased cytotoxicity. This is achieved by assaying the cells for cytotoxicity after each dose of radiation has been administered.

There are significant benefits of using an irradiated NK cell line for adoptive cellular immunotherapy over the well-established autologous or MHC-matched T cell approach. Firstly, the use of a NK cell line with a highly proliferative nature means expansion of modified NK cell lines can be achieved more easily and on a commercial level. Irradiation of the modified NK cell line can then be carried out prior to administration of the cells to the patient. These irradiated cells, which retain their useful cytotoxicity, have a limited life span and, unlike modified T cells, will not circulate for long periods of time causing persistent side-effects.

Additionally, the use of allogeneic modified NK cells and NK cell lines means that MHC class I expressing cells in the patient are unable to inhibit NK cytotoxic responses in the same way as they can to autologous NK cytotoxic responses. The use of allogeneic NK cells and cell lines for cancer cell killing benefits from the previously mentioned GVL effect and, unlike for T cells, allogeneic NK cells and cell lines do not stimulate the onset of GVHD, making them a preferred option for the treatment of cancer via adoptive cellular immunotherapy.

### Examples

The present invention is now described in more and specific details in relation to the production of NK cells that are modified to exhibit more cytotoxic activity against a range of cancer cells.

For related examples of knockout / knockdown of inhibitory receptor function and knock in of TRAIL / mutant TRAIL we refer to WO 2017/017184, the contents of which are incorporated herein by reference.

For related examples of CD38 CAR expression we refer to WO 2018/104562, the contents of which are incorporated herein by reference.

For related examples of MUC-1 CAR expression we refer to WO 2019/101998, the contents of which are incorporated herein by reference.

The invention is now illustrated in specific embodiments with reference to the accompanying drawings in which:

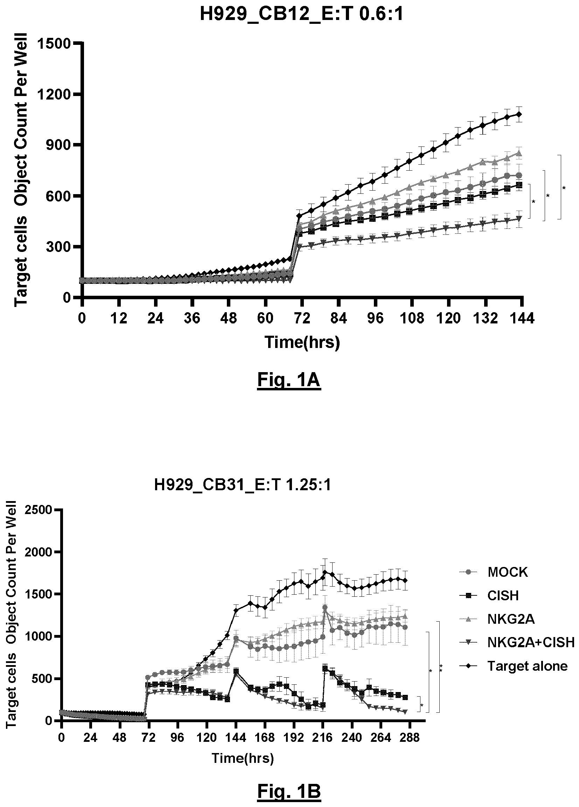

Fig. 1A shows a serial killing assay of H929-RFP-Luc cells by CB-derived NK cells from "Donor 1" (CB12) that are unmodified (mock), have a CISH KO, have a NKG2A KO, or have a CISH/NKG2A DKO. Each data point is measured in duplicate. Statistical significance is determined via the unpaired t test on data from the final time point only. *= p<0.05 and ** = p<0.01;
Fig. 1B shows a serial killing assay of H929-RFP-Luc cells by CB-derived NK cells from "Donor 2" (CB31) that are unmodified (mock), have a CISH KO, have a NKG2A KO, or have a CISH/NKG2A DKO. Each data point is measured in duplicate. Statistical significance is determined via the unpaired t test on data from the final time point only. *= p<0.05 and ** = p<0.01;

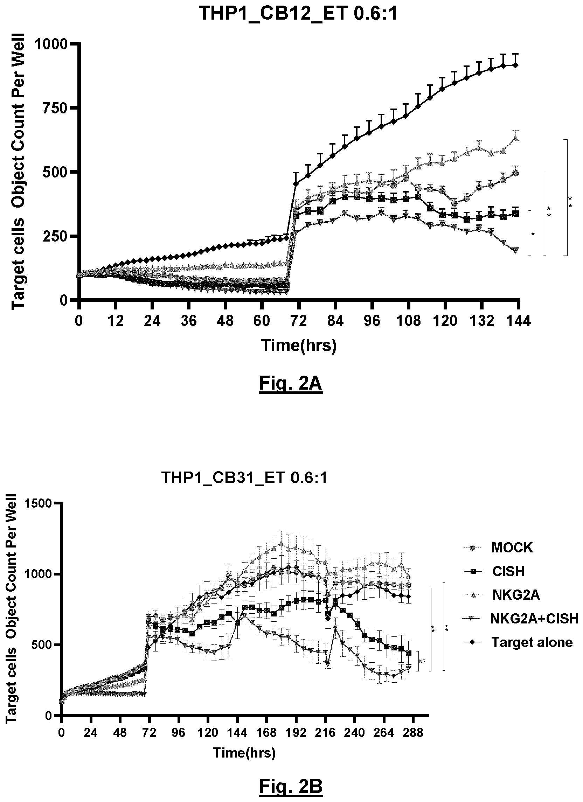

Fig. 2A shows a serial killing assay of THP1-RFP-Luc cells by CB-derived NK cells from "Donor 1" (CB12) that are unmodified (mock), have a CISH KO, have a NKG2A KO, or have a CISH/NKG2A DKO. Each data point is measured in duplicate. Statistical significance is determined via the unpaired t test on data from the final time point only. *= p<0.05 and ** = p<0.01; and
Fig. 2B shows a serial killing assay of THP1-GFP-Luc cells by CB-derived NK cells from "Donor 2" (CB31) that are unmodified (mock), have a CISH KO, have a NKG2A KO, or have a CISH/NKG2A DKO. Each data point is measured in duplicate. Statistical significance is determined via the unpaired t test on data from the final time point only. *= p<0.05 and ** = p<0.01.

### Methods

### Production of gene-edited cord blood (CB)-derived NK cells

Mononuclear cells were isolated from fresh cord isolates derived from two donors (CB12 and CB31), CD3 depleted and cryopreserved in Cryostor 10 (CS10) medium in CoolCell containers at -80°C before transfer to -150°C for long-term storage. Cells were later rapidly thawed at 37°C, washed and activated using irradiated EBV-LCL feeder cells at a 10:1 ratio of feeder cells to total viable MNCs in NK MACS culture medium supplemented with 10% human AB serum and 500 IU/mL recombinant human IL2 (rh IL2). All cell culture was performed using GRex 24 or 6 well containers at 37°C / 5% CO₂. After 7 days in culture, cells were electroporated (MaxCyte) with ribonucleic acid-protein (RNP) complexes consisting of Cas9 + CRISPR guide RNA targeting either CISH or NKG2A alone, or CISH + NKG2A together. Control cells underwent a mock electroporation consisting of cells alone. After electroporation, cells were incubated at 30°C for 24h followed by a second feeder cell addition at a 2:1 ratio of feeder cells to total viable MNCs. Cells were then cultured for 14 days after editing and frozen in CS10 medium in CoolCell containers at -80°C before transfer to -150 °C for long term storage.

### Serial killing assay of gene-edited cord blood (CB)-derived NK cells

Cryopreserved mock or gene edited CB-derived NK cells were rapidly thawed at 37°C, washed and recovered for 24h in 500 IU/mL rh IL2. These cells were then co-cultured with either H929-RFP-Luc or THP1-GFP-Luc cells (multiple myeloma cells and leukaemia cells, respectively) at indicated E:T ratios for 72h in the absence of IL2 at 37°C / 5% CO₂. Tumour cell growth was determined by Incucyte measurement of object cells positive for GFP over time with readings taken every 12-24h. Fresh tumour cells and recombinant human IL15 (rh IL15) (5ng/mL) were added at 72h, 144h and 216h. The number of tumour cells added at each time point matched the original numbers added at 0h.

### Results

### CISH / NKG2A Knockout

At 14 days post-electroporation, mean NKG2A KO, as measured by surface protein detection by flow cytometry, was 60% +/- 15% in the single KO cells and 56% +/-17% in the DKO cells.

Mean CISH KO, as measured by Sanger sequencing, was 75% +/- 6% in single KO cells and 80% +/- 6% in double KO cells.

### Serial cytotoxicity against tumour cell lines

Against the H929-RFP-Luc multiple myeloma (MM) cells, mock electroporated CB-derived NK cells mediated a low level of cytotoxicity compared to target cells cultured alone. CB-derived NK cells with a single NKG2A KO performed similarly or worse than their mock counterparts. Single CISH KO CB NK cells showed some evidence of improved killing particularly in Donor 2 (see Fig. 1B), however NKG2A and CISH double knockout (DKO) CB-NK cells mediated significantly improved killing compared to other conditions (Fig 1A and 1B). This improved killing is synergistic (not additive) due to the poor performance of CB-NK cells that were NKG2A KO alone.

Against the THP1-GFP-Luc leukaemia cells, mock electroporated CB-derived NK cells mediated a relatively low level of cytotoxicity compared to target cells alone. CB-NK cells with a single NKG2A KO performed similarly or worse than their mock counterparts. Single CISH KO CB-NK cells demonstrated improved killing compared to mock or NKG2A KO cells in both donors, however NKG2A and CISH double knockout (DKO) CB-NK cells mediated significantly improved killing compared to each of these conditions in Donor 1 and in Donor 2 CB-NK cells (Fig 2A and 2B). This improved killing is synergistic (not additive) due to the poor performance of CB-NK cells that were NKG2A KO alone.

The invention thus provides NK cells and NK cell lines with increased cytotoxicity, as well as uses of these cells in cancer therapy.

## Claims

1. A natural killer (NK) cell or NK cell line that has been genetically modified to have reduced expression of both CISH and NKG2A.

2. An NK cell or NK cell line according to claim 1, wherein the modification is to knock down or knock out expression of both CISH and NKG2A.

3. An NK cell or NK cell line according to any preceding claim, wherein CISH expression is reduced by at least 50% compared to the same NK cell or NK cell line without the modification.

4. An NK cell or NK cell line according to any preceding claim, wherein CISH expression is reduced by at least 90% compared to the same NK cell or NK cell line without the modification.

5. An NK cell or NK cell line according to any preceding claim, wherein NKG2A expression is reduced by at least 50% compared to the same NK cell or NK cell line without the modification.

6. An NK cell or NK cell line according to any preceding claim, wherein NKG2A expression is reduced by at least 90% compared to the same NK cell or NK cell line without the modification.

7. An NK cell or NK cell line according to any preceding claim, wherein expression of both CISH and NKG2A is knocked out.

8. An NK cell or NK cell line according to any preceding claim, further modified to express IL-15.

9. An NK cell or NK cell line according to any preceding claim, further modified to express wildtype TRAIL or a TRAIL variant with increased affinity for TRAIL death receptors.

10. An NK cell or NK cell line according to any preceding claim, further modified to express a chimeric antigen receptor (CAR) for HER2, TIM-3, CD19, CD38, MUC-1, CLL-1, SLAMF7 and/or CD96.

11. An NK cell or NK cell line according to any preceding claim, wherein the NK cell or NK cell line has no additional modifications to inhibitory receptor expression.

12. An NK cell or NK cell line according to any preceding claim, wherein the NK cell or NK cell line expresses both TIGIT and TGFBRII.

13. An NK cell or NK cell line according to any preceding claim, for use in treating cancer.

14. An NK cell or NK cell line for use according to claim 13, wherein the cancer is an HLA-E expressing cancer.

15. An NK cell or NK cell line for use according to claim 13 or 14, wherein the cancer is selected from multiple myeloma, acute myeloid leukaemia and acute lymphoblastic leukaemia.
